(19) Europäisches Patentamt
European Patent Office
Office européen des brevets

(11) Publication number: 0 075 019 B1

(12) # EUROPEAN PATENT SPECIFICATION

(45) Date of publication of patent specification: 21.11.85

(51) Int. Cl.⁴: C 07 D 209/08

(21) Application number: 81900753.5

(22) Date of filing: 25.03.81

(86) International application number: PCT/JP81/00064

(87) International publication number: WO 82/03391 14.10.82 Gazette 82/25

(54) ## PROCESS FOR PREPARING INDOLE OR INDOLE DERIVATIVES.

(43) Date of publication of application: 30.03.83 Bulletin 83/13

(45) Publication of the grant of the patent: 21.11.85 Bulletin 85/47

(84) Designated Contracting States: CH DE FR GB LI NL

(56) References cited:
WO-A-82/00032
JP-A-55 105 663

W.C.SUMPTER et al.: "Heterocyclic compounds with INDOLE and CARBAZOLE SYSTEMS", The Chemistry of Heterocyclic Compounds, 1954 Interscience Publishers, Inc., New York, pages 20-21

PATENTS ABSTRACTS OF JAPAN, vol. 5, no. 94 (C-59) (766), June 19, 1981

PATENTS ABSTRACTS OF JAPAN, vol. 5, no. 106 (C-62) (778), July 10, 1981

PATENTS ABSTRACTS OF JAPAN, vol. 5, no. 114 (C64) (786), July 23, 1981

(73) Proprietor: MITSUI TOATSU CHEMICALS, INCORPORATED
2-5 Kasumigaseki 3-chome
Chiyoda-Ku Tokyo 100 (JP)

(72) Inventor: MATSUDA, Fujio
1965-12, Fueta Kamakura-shi
Kanagawa 248 (JP)
Inventor: KATOU, Takazou
544, Kamiohi Ohimachi
Ashigarakami-gun Kanagawa 258 (JP)
Inventor: HONDA, Tadatoshi
8-4, Fujimino 2-chome
Hiratsuka-shi Kanagawa 259-12 (JP)
Inventor: TERADA, Kazuhiro
41-9, Tobehoncho Nishi-ku
Yokohama-shi Kanagawa 220 (JP)
Inventor: KOGURE, Yasuo
1-3-205, Maborikaigan 4-chome
Yokosuka-shi Kanagawa 239 (JP)
Inventor: KIYOURA, Tadamitsu
1471, Kajiwara
Kamakura-shi Kanagawa 247 (JP)

(74) Representative: Paget, Hugh Charles Edward et al
MEWBURN ELLIS & CO. 2/3 Cursitor Street
London EC4A 1BQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 075 019 B1

Courier Press, Leamington Spa, England.

(56) References cited:

PATENTS ABSTRACTS OF JAPAN, vol. 5, no. 114, (C-64) (786), July 23, 1981

PATENTS ABSTRACTS OF JAPAN, vol. 5, no. 128 (C-67) (800), August 18, 1981

PATENTS ABSTRACTS OF JAPAN, vol. 5, no. 140 (C-70) (812), September 4, 1981

PATENTS ABSTRACTS OF JAPAN, vol. 5, no. 156 (C-74) (828), October 6, 1981

PATENTS ABSTRACTS OF JAPAN, vol. 5, no. 188 (C-81) (860), November 27, 1981

PATENTS ABSTRACTS OF JAPAN, vol. 6, no. 31 (C-92) (909), February 24, 1982

PATENTS ABSTRACTS OF JAPAN, vol. 6, no. 57 (C-98) (935), April 14, 1982

PATENTS ABSTRACTS OF JAPAN, vol. 5, no. 194 (C-82) (866), December 10, 1981

**Description**

Technical field

This invention relates to a novel process for the preparation of indole and derivatives thereof by reacting an aniline with a specific 1,2-glycol or an ethanolamine.

Background art

In the prior art, indole derivatives have long been prepared by the well-known Fischer indole synthesis in which phenylhydrazine is reacted with a compound having an aldehyde group. If the aldehyde compound is other than acetaldehye, the aforesaid Fischer indole synthesis can be applied to obtain indole derivatives in good yield. However, if the alehyde compound is acetaldehyde, no reaction that yields indole has been believed to take place. In order to overcome this disadvantage, there has recently been proposed an improved process which comprises reacting phenylhydrazine with acetaldehyde at an elevated temperature of from 300° to 400°C in the presence of an alumina catalyst (Japanese Patent Laid-Open No. 76864/1973).

This process really permits the reaction to proceed and brings about the formulation of indole, but fails to give a satisfactory yield. Moreover, it is greatly disadvantageous in that the catalyst has so short a life as to become totally inactive after 0.5-1 hour's use.

Indole can also be prepared by another process which comprises reacting *o*-toluidine with formic acid to form *o*-methyl-N-formylaniline and then fusing it together with potassium hydroxide. However, it is usually impossible to selectively prepare *o*-toluidine that is used as the starting material in this process. That is, the *p*-isomer is always formed in an amount equal to or greater than that of the *o*-isomer. Thus, treatment of the isomer formed as a by-product poses a serious problem in the case of industrial production. Moreover, the handling of solids as in alkali fusion is troublesome. For these reasons, the aforesaid process cannot be regarded as suitable for industrial purposes.

Furthermore, a number of attempts have been made to synthesize indole from N-ß-hydroxyethylamine, but none of them are satisfactory from an industrial point of view. For example, a process which comprises effecting the reaction at 300°C in the presence of aluminosilicate catalyst [Zhur. obschue. Khim., Vol. 24, pp. 671-678 (1954)] gives only a very low yield of indole. A process which comprises heating the reactant together with a molten mixed salt consisting mainly of zinc chloride (Japanese Patent Laid-Open No. 57968/1973) can give a fairly high yield of indole. However, this process has the disadvantage of requiring a complicated procedure, which makes it unsuitable for industrial purposes.

In 1883, Fischer and German (Ber., *16,* 710-712) investigated the reaction of aniline, glycerol and zinc chloride. A trace of skatole (1.5% based on aniline) was obtained.

As described above, a number of processes for the synthesis of indole and derivatives thereof have been proposed. However, many of them are disadvantageous in that large amounts of by-products are formed, expensive compounds are used as the starting materials, and/or lengthy and complicated procedures are required to obtain the desired products.

It is an object of the present invention to provide a one-step process for the highly selective preparation of indole and derivatives thereof by using inexpensive compounds as the starting materials.

According to the present invention, there is provided a process for the preparation of indole and derivatives thereof which comprises reacting an aniline with a 1,2-glycol (or equivalent) as specified below, or an ethanolamine.

This reaction can be carried out both in the liquid phase and in the vapor phase. By way of example, the process of the present invention makes it possible to obtain indole by reacting aniline with ethylene glycol and to obtain 5-methylindole by reacting *p*-toluidine with ethylene gylcol. Moreover, the process also makes it possible to obtain indole by reacting aniline with an ethanolamine and to obtain 5-methylindole by reacting *p*-toluidine with an ethanolamine.

Thus, the process of the present invention has a number of advantages. First, the anilines, 1,2-glycols and ethanolamines which can be used as the starting materials may be very inexpensive. Secondly, the preparation of indole or a derivative thereof from the starting materials can be achieved in a single step. Thirdly, by-products are scarcely formed and a very high selectivity is attained, so that indole or a derivative thereof can be obtained in a highly pure form.

The aniline used in the process of the present invention may be a compound of the general formula

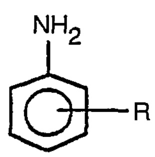

(I)

wherein R represents a hydrogen atom, halogen atom, hydroxyl group, alky group or alkoxy group. Specific examples thereof are aniline, *o*-toluidine, *m*-toluidine, *p*-toluidine, *o*-haloanilines, *p*-haloanilines, *m*-haloanilines, *o*-aminophenol, *m*-aminophenol, *p*-aminophenol, *o*-anisidine, *m*-anisidine, and *p*-anisidine.

The 1,2-glycol or equivalent used in the process of the present invention is selected from: ethylene glycol, propylene glycol, 1,2-butanediol, 2,3-butanediol, and diethylene glycol.

The ethanolamine used in the process of the present invention may be selected from monoethanolamine, diethanolamine, and triethanolamine.

Although the process of the present invention can be carried out in the absence of catalyst, it is preferably carried out in the presence of a solid acid catalyst, a metallic catalyst or activated carbon to obtain the desired product in good yield.

The solid acid catalysts which can be used in the process of the present invention fall under the following three categories:

(1) Catalysts containing an oxide or hydroxide (hereinafter referred to as the catalytic substance (1)) of at least one element selected from the group consisting of Si, Al, B, Sb, Bi, Sn, Pb, Ga, Ti, Zr, Be, Mg, Y, Cu, Ag, Zn, Cd and the lanthanides. Specific examples of the catalytic substance (1) are CdO, $ZnO$-$Sb_2O$, $PbO_2$, $Al_2O_3$-$B_2O_3$, $SiO_2$-CdO, $SiO_2$-$Al_2O_3$, $SiO_2$-MgO, $TiO_2$-$SnO_2$, $TiO_2$-$ZrO_2$, CdO-$Bi_2O_3$, $SiO_2$-$Y_2O_3$, $SiO_2$, $Bi_2O_3$-BeO, $SiO_2$-$La_2O_3$, $SiO_2$-$Ce_2O_3$, $SiO_2$-ZnO-AgO, $SiO_2$-MgO-CuO, $SiO_2$-$Ga_2O_3$.

(2) Catalysts containing a sulfide or selenide (hereinafter referred to as the catalytic substance (2)) of at least one element selected from the group consisting of Pd, Pt, Cr, Fe, Ni, Co, Zn, Mo, Cd and W. Specific examples of the catalytic substance (2) are PdS, PtS, CrS, FeS, NiS, CoS, ZnS, $MoS_2$, CdS, $WS_2$, ZnSe, CdSe.

(3) Catalysts containing an inorganic acid salt (hereinafter referred to as the catalytic substance (3)) of at least one element selected from the group consisting of Fe, Tl, Ca, Mn, Bi, Sr, Y, Al, Zn, Cd, Ni, Mg, In, Be, Co, Ga and the lanthanides. The useful inorganic acid salts include halides, carbonates, nitrates, sulfates, phosphates, pyrophosphates, phosphomolybdates and silicotungstates, and specific examples of the catalytic substance (3) are ferric sulfate, thallium sulfate, calcium sulfate, manganese sulfate, bismuth sulfate, strontium sulfate, yttrium sulfate, cadmium bromide, aluminum sulfate, zinc sulfate, nickel sulfate, cadmium chloride, magnesium sulfate, indium sulfate, beryllium sulfate, cadmium nitrate, cobalt sulfate, zinc aluminum sulfate, magnesium chloride, cadmium sulfate, cadmium phosphate.

The metallic catalysts which can be used in the process of the present invention include catalysts containing at least one element (hereinafter referred to as the catalytic substance (4)) selected from the group consisting of Cu, Ag, Pt, Pd, Ni, Co, Fe, Ir, Os, Ru and Rh.

The solid acid catalysts and metallic catalysts which are usable in the process of the present invention can be prepared by any suitable methods that are known in this field of art. More specifically, solid acid catalysts falling under the category of the catalytic substance (1) can be prepared, for example, by hydrolyzing a water-soluble salt of the principal constituent element of the desired catalyst to form its hydroxide and then drying and calcining the resulting gel, or by pyrolyzing an easily decomposable salt of the principal constituent element of the desired catalyst in air.

Solid acid catalysts falling under the category of the catalytic substance (2) can be prepared, for example, by adding sodium sulfide or potassium selenide to a water-soluble salt of the principal constituent element of the desired catalyst or by contacting the principal constituent element of the desired catalyst or a salt thereof with hydrogen sulfide gas or hydrogen selenide gas.

Metallic catalysts falling under the category of the catalytic substance (4) can be prepared, for example, by reducing a salt, hydroxide or oxide of the principal constituent element of the desired catalyst by means of a reducing agent such as hydrogen, formalin, formic acid, phosphorous acid, or hydrazine.

In the process of the present invention, the above-described catalytic substances (1), (2), (3) and (4) may be used alone or in admixture. Moreover, these catalytic substances and mixtures thereof may be used as such or in the form of supported catalysts. Although any carriers that are in common use for this purpose can be used, diatomaceous earth, pumice, titania, silica-alumina, alumina, magnesia, silica gel, activated carbon, activated clay, asbestos are used in typical cases. Supported catalysts can be prepared by supporting the above-described catalytic substances on these carriers according to any conventional techniques. No particular limitation is placed on the amount of catalytic substance supported on the carrier. Usually, depending on the type of carrier used, any suitable amount (for example, from 1 to 50%) of catalytic substance may be supported thereon.

In addition, various types of activated carbon can be used in the process of the present invention. They include, for example, products made from coconut shell, wood, sawdust, lignin, coal, blood charcoal, bone charcoal, petroleum carbon. They are commercially available in powdered form, in crushed form, or in shaped form (for example, in the shape of globules or cylinders). However, no particular limitation is placed on the form of activated carbon used.

Among the solid acid catalysts falling under the category of the catalytic substance (3), the sulfates and particularly cadmium sulfate are preferred for the purpose of obtaining the desired product in good yield. Among the solid acid catalysts falling under the category of the catalytic substance (2), cadmium sulfide and cadmium selenide are particularly preferred. Among the metallic catalysts falling under the category of the catalytic substance (4), Ag is preferred.

Although the process of the present invention can be carried out in the vapor phase, the liquid phase or a mixed vapor-liquid phase, it is usually carried out in the vapor phase. Where the process of the present invention is carried out in the vapor phase, a fixed-bed, fluidized-bed or moving-bed reactor can be used to effect the reaction by heating the vapors of an aniline and a 1,2-glycol or an ethanolamine in the presence or absence of a catalyst. In this case, various inert gaseous substances may coexist as diluents for the

vapors of the starting materials. The useful inert gaseous substances include, for example, nitrogen gas, carbon dioxide gas, water vapor, and the vapors of compounds that are inert to this reaction. Moreover, hydrogen gas or a hydrogen-containing gas may be used as a diluent.

The use of hydrogen gas or a hydrogen-containing gas is especially suitable for the purpose of maintaining the activity of the catalyst.

Similarly, owing to its ability to suppress the decomposition of the 1,2-glycol or ethanolamine over the catalyst, the use of water vapor is suitable for the purpose of maintaining the activity of the catalyst and enhancing the yield of the desired product.

The amounts of aniline and 1,2-glycol fed to the reactor should be such that from 0.01 to 5 moles and preferably from 0.05 to 2 moles of the 1,2-glycol or ethanolamine is provided for each mole of the aniline. If the amounts are outside this range, a reduction in yield will be caused and/or large amounts of by-products will be formed. These starting materials are fed, after being vaporized in advance or directly in liquid form, to the reactor at a liquid space velocity of from 0.01 to 5 liters/liter of the catalyst/hour.

The process of the present invention is carried out at a reaction temperature in the range of from 200° to 600°C and preferably from 250° to 500°C. If the reaction temperature is lower than 200°C, the reaction can hardly proceed, while if it is higher than 600°C, undesirably large amounts of by-products will be formed.

The reaction pressure may be superatmospheric, atmospheric or subatmospheric.

Where the process of the present invention is carried out in the liquid phase or a mixed vapor-liquid phase, the reaction is effected by heating a mixture of an aniline and a 1,2-glycol or an ethanolamine in the presence of at least one member selected from the above-described catalysts. In this case, various inert gaseous substances and/or solvents may coexist as diluents for the starting materials. The useful inert gaseous substances include, for example, nitrogen gas, carbon dioxide gas, water vapor and the vapors of compounds that are inert to this reaction. The useful solvents include, for example, benzene, toluene, xylene, methanol, ethanol, isopropanol, dioxane, dimethylformamide, dimethyl sulfoxide, pyridine, N-methylpyrrolidone, trimethylamine, diethylamine, triethylamine, tripropylamine, tributylamine, diphenylamine, triphenylamine and other organic solvents.

In the case of liquid-phase reaction, the process of the present invention can be carried out in a fixed-bed, fluidized-bed or moving-bed reactor or in a rotary of continuous reactor for liquid-phase reactions. However, no particular limitation is placed on the type of reactor used.

The amounts of aniline and 1,2-glycol or ethanolamine used as the starting materials for this reaction should be such that from 0.05 to 5 moles and preferably from 0.1 to 2 moles of 1,2-glycol or ethanolamine is provided for each mole of the aniline.

No particular limitation is placed on the amount of catalyst used for this reaction. However, the catalyst is generally used in an amount of from 0.01 to 20 g and preferably from 0.1 to 10 g of the active component thereof per mole of the aniline used as one of the starting materials.

The reaction temperature should be in the range of from 200° to 500°C and preferably from 250° to 400°C. If the reaction temperature is lower than 200°C, the reaction can hardly proceed, while if it is higher than 500°C, undesirably large amounts of by-products will be formed.

The reaction pressure may be superatmospheric or atmospheric.

In various embodiments of the present invention, indole or a derivative thereof can readily be obtained in pure form by isolating it from the reaction product according to any conventional technique such as distillation.

The present invention is further illustrated by the following examples.

## Example 1

Cadmium sulfide in powder form was compressed and then crushed to particles. A 10-mm glass flow reactor made of Pyrex was packed with 5 ml of the particles. The front end of this reactor was connected with a feed inlet pipe and a gas inlet pipe to form a feed vaporization zone, while the rear end thereof was connected with a receiver by way of an air-cooling zone.

In the reaction zone, the internal temperature of the reactor was kept at 325°C. Then, a mixture consisting of 1 mole of aniline and 0.2 mole of ethylene glycol was introduced thereinto through the feed inlet pipe at a liquid space velocity of 0.1 liter/liter of the catalyst/hour. At the same time, nitrogen gas at atmospheric pressure was passed therethrough in an amount of 10 moles per mole of the aniline used as one of the starting materials. After the reaction was carried out for 3 hours, the reaction product withdrawn from the reactor, condensed and collected in the receiver was analyzed by gas chromatography. This revealed that indole was obtained in a 59% yield based on the ethylene glycol and accompanied with very small amounts of by-products.

## Example 2

The procedure of Example 1 was repeated by using a variety of catalysts. The results thus obtained are summarized in Table 1.

TABLE 1

| Run No. | Catalyst: Type | Composition (weight ratio) | Amount Supported on Carrier (% by weight) | Yield of Indole (%) |
|---|---|---|---|---|
| 1 | Cdo | — | — | 20 |
| 2 | $Al_2O_3$-$B_2O_3$ | 5:1 | — | 10 |
| 3 | $SiO_2$-CdO | 1:1 | — | 18 |
| 4 | $SiO_2$-ZnO | 1:1 | — | 19 |
| 5 | Zeolite 13X | — | — | 30 |
| 6 | $SiO_2$-MgO | 1:1 | — | 18 |
| 7 | $TiO_2$-$SnO_2$ | 1:1 | — | 13 |
| 8 | CdO-$Bi_2O_3$ | 1:1 | — | 15 |
| 9 | $SiO_2$-$Y_2O_3$ | 1:1 | — | 16 |
| 10 | $SiO_2$ | — | — | 15 |
| 11 | $Bi_2O_3$-$B_2O$ | 1:1 | — | 12 |
| 12 | $SiO_2$-$La_2O_3$ | 1:1 | — | 11 |
| 13 | $SiO_2$-$Ce_2O_3$ | 1:1 | — | 12 |
| 14 | $SiO_2$-ZnO-AgO | 1:1:1 | — | 40 |
| 15 | $SiO_2$-MgO-CuO | 2:1:1 | — | 20 |
| 16 | PdS/C | — | 1 | 16 |
| 17 | PtS/C | — | 1 | 25 |
| 18 | FeS | — | — | 38 |
| 19 | ZnS | — | — | 30 |
| 20 | $MoS_2$ | — | — | 12 |
| 21 | ZnSe | — | — | 15 |
| 22 | CdSe | — | — | 52 |
| 23 | $Tl_2SO_4$ | — | — | 10 |
| 24 | $MnSO_4 \cdot 4—6H_2O$ | — | — | 11 |
| 25 | $SrSO_4$ | — | — | 12 |
| 26 | $Y_2(SO_4)_3 \cdot 8H_2O$ | — | — | 13 |
| 27 | $Al_2(SO_4)_3 \cdot 16—18H_2O$ | — | — | 12 |
| 28 | $ZnSO_4 \cdot 7H_2O$ | — | — | 15 |

TABLE 1 (continued)

| Run No. | Catalyst | | | Yield of Indole (%) |
|---|---|---|---|---|
| | Type | Composition (weight ratio) | Amount Supported on Carrier (% by weight) | |
| 29 | $NiSO_4 \cdot 6H_2O$ | — | — | 20 |
| 30 | $CdCl_2 \cdot \frac{1}{2}H_2O$ | — | — | 22 |
| 31 | $MgSO_4 \cdot 7H_2O$ | — | — | 26 |
| 32 | $In_2(SO_4)_3$ | — | — | 28 |
| 33 | $BeSO_4 \cdot 4H_2O$ | — | — | 38 |
| 34 | $CdSO_4 \cdot 4H_2O$ | — | — | 91 |
| 35 | $CoSO_4 \cdot 7H_2O$ | — | — | 40 |
| 36 | $MgCl_2 \cdot 6H_2O$ | — | — | 42 |
| 37 | $Cd(NO_3)_2$ | — | — | 16 |
| 38 | $Cd_3(PO_4)_2 \cdot 2CdHPO_4$ | — | — | 12 |
| 39 | Raney copper catalyst | — | — | 15 |
| 40 | Al-α-$Al_2O_3$ carrier | — | 10 | 50 |
| 41 | Pt-activated carbon carrier | — | 5 | 36 |
| 42 | Pd-activated carbon carrier | — | 0.5 | 42 |
| 43 | Raney nickel catalyst | — | — | 27 |
| 44 | Raney cobalt catalyst | — | — | 22 |
| 45 | Reduced iron | — | — | 18 |
| 46 | Ir-asbestos carrier | — | 5 | 32 |
| 47 | Os-activated carbon carrier | — | 5 | 34 |
| 48 | Ru-alumina carrier | — | 5 | 38 |
| 49 | Rh-alumina carrier | — | 5 | 43 |
| 50 | Granular activated carbon | — | — | 21 |

## Example 3

The procedure of Example 1 was repeated except that 5 ml of glass beads having a diameter of 2 mm was used in place of the catalyst of Example 1. As a result, indole was obtained in a 1% yield. Then, the same procedure was repeated once more at a reaction temperature of 500°C to obtain indole in a 6% yield.

## Example 4

Using the catalyst of Example 1, reaction was carried out for 27 hours in the same manner as described in Example 1. The reaction mixture collected between the start of the reaction and 3 hours after that (hereinafter referred to as reaction mixture A) and the reaction mixture collected between 24 hours and 27 hours after the start of the reaction (hereinafter referred to as reaction mixture B) were analyzed. This revealed that the yield of indole was 58% for reaction mixture A and 25% for reaction mixture B.

Example 5

The procedure of Example 4 was repeated except that hydrogen gas was used in place of the nitrogen gas. This revealed that the yield of indole was 60% for reaction mixture A and 48% for reaction mixture B.

Example 6

The procedure of Example 4 was repeated except that a mixed gas consisting of hydrogen gas and water vapor in a molar ratio of 9:1 was used in place of the nitrogen gas. This revealed that the yield of indole increased to 69% for reaction mixture A and 65% for reaction mixture B, thus showing only a slight difference therebetween.

Example 7

The procedures of Examples 4, 5 and 6 were repeated by using a catalyst comprising 10% by weight of Ag supported on an $SiO_2$-ZnO carrier (in a weight ratio of 1:1). The results thus obtained are summarized in Table 2.

TABLE 2

| Run No. | Gas Used | | Yield of Indole | |
|---|---|---|---|---|
| | Type | Molar Ratio | Reaction Mixture A | Reaction Mixture B |
| 51 | Nitrogen | — | 61% | 30% |
| 52 | Hydrogen | — | 62% | 50% |
| 53 | Hydrogen + water vapor | 9:1 | 71% | 68% |

Example 8

The procedure of Example 1 was repeated except that cadmium sulfate was used as the catalyst and the compounds indicated in Table 3 were used as the starting materials fed to the reactor. The results thus obtained are summarized in Table 3.

TABLE 3

| Run No. | Starting Materials | | Results | |
|---|---|---|---|---|
| | Aniline | 1,2-Glycol | Product | Yield |
| 54 | *p*-Toluidine | Ethylene glycol | 5-Methylindole | 40% |
| 55 | *p*-Chloroaniline | Ethylene glycol | 5-Chloroindole | 70% |
| 56 | *o*-Anisidine | Ethylene glycol | 7-Methoxyindole | 32% |
| 57 | Aniline | Propylene glycol | Skatole | 90% |
| 58 | Aniline | Monoethanolamine | Indole | 83% |
| 59 | Aniline | Diethanolamine | Indole | 76% |

Example 9

Into a 200-ml autoclave made of a titanium alloy and fitted with a stirrer was charged with 93.1 g (1 mole) of aniline, 12.4 g (0.2 mole) of ethylene glycol, and 2 g of a palladium-carbon catalyst in powder form (having a Pd content of 0.5% by weight). After the autoclave was purged with nitrogen gas and filled therewith to a pressure of 5 kg/$cm^2$, reaction was carried out at 300°C for 30 minutes with stirring. After completion of the reaction, the catalyst was separated from the reaction mixture by filtration and the resulting reaction product was analyzed by gas chromatography. This revealed that indole was obtained in a 38% yield based on the ethylene glycol.

Example 10

The procedure of Example 9 was repeated except that 1 g of magnesium oxide was used in place of the palladium-carbon catalyst. After completion of the reaction, the magnesium oxide was separated from the reaction mixture by filtration and the resulting reaction product was analyzed by gas chromatography. This revealed that indole was obtained in a 60.7% yield based on the ethylene glycol and accompanied with a small amount of indoline formed as a by-product.

**Claims**

1. A process for the preparation of indole and derivatives thereof which comprises reacting an aniline with a compound selected from: ethanolamines; ethylene glycol; propylene glycol; 1,2-butanediol; 2,3-butanediol; diethylene glycol.

2. A process as claimed in claim 1 wherein the reaction is carried out in the presence of a solid acid catalyst, a metallic catalyst or activated carbon.

3. A process as claimed in claim 1 or 2 wherein the reaction is carried out in the vapor phase at a temperature of from 200° to 600°C.

4. A process as claimed in claim 1 or 2 wherein the reaction is carried out in the liquid phase or a mixed vapor-liquid phase at a temperature of from 200° to 500°C.

5. A process as claimed in claim 1 or 2 wherein the reaction is carried out in an atmosphere of hydrogen gas or a hydrogen-containing gas.

6. A process as claimed in claim 1 or 2 wherein the reaction is carried out in the presence of water or water vapor.

7. A process as claimed in claim 2 wherein the solid acid catalyst is a catalyst containing an oxide or hydroxide of at least one element selected from the group consisting of Si, Al, B, Sb, Bi, Sn, Pb, Ga, Ti, Zr, Be, Mg, Y, Cu, Ag, Zn, Cd and the lanthanides.

8. A process as claimed in claim 2 wherein the solid acid catalyst is a catalyst containing a sulfide or selenide of at least one element selected from the group consisting of Pd, Pt, Cr, Fe, Ni, Co, Zn, Mo, Cd and W.

9. A process as claimed in claim 2 wherein the solid acid catalyst is a catalyst containing an inorganic acid salt of at least one element selected from the group consisting of Fe, Tl, Ca, Mn, Bi, Sr, Y, Al, Zn, Cd, Ni, Mg, In, Be, Co, Ga and the lanthanides.

10. A process as claimed in claim 2 wherein the metallic catalyst is a catalyst containing at least one element selected from the group consisting of Cu, Ag, Pt, Ni, Co, Fe, Ir, Os, Ru and Rh.

11. A process as claimed in claim 9 wherein the inorganic acid salt is a sulfate.

12. A process as claimed in claim 8 or 11 wherein one of the constituent elements of the catalyst is Cd.

13. A process as claimed in claim 10 wherein one of the constituent elements of the catalyst is Ag.

**Revendications**

1. Procédé de préparation d'indole et ses dérivés qui consiste à faire réagir une aniline avec un composé choisi parmi: éthanolamines; éthylène glycol; propylène glycol; 1,2-butanediol; 2,3-butanediol; diéthylène glycol.

2. Procédé selon la revendication 1 où la réaction est effectuée en présence d'un catalyseur acide solide, d'un catalyseur métallique ou de charbon activé.

3. Procédé selon la revendication 1 ou la revendication 2, où la réaction est effectuée en phase vapeur et à une température de 200 à 600°C.

4. Procédé selon la revendication 1 ou 2, où la réaction est effectuée en phase liquide ou en phase liquide-vapeur mélangée à une température de 200 à 500°C.

5. Procédé selon la revendication 1 ou 2, où la réaction est effectuée dans une atmosphère d'hydrogène gazeux ou d'un gaz contenant de l'hydrogène.

6. Procédé selon la revendication 1 ou 2, où la réaction est effectuée en présence d'eau ou de vapeur d'eau.

7. Procédé selon la revendication 2, où le catalyseur acide solide est un catalyseur contenant un oxyde ou hydroxyde d'au moins un élément choisi dans le groupe consistant en Si, Al, B, Sb, Bi, Sn, Pb, Ga, Ti, Zr, Be, Mg, Y, Cu, Ag, Zn, Cd et les lanthanides.

8. Procédé selon la revendication 2, où le catalyseur acide solide est un catalyseur contenant un sulfure ou un séléniure d'au moins un élément choisi dans le groupe consistant en Pd, Pt, Cr, Fe, Ni, Co, Zn, Mo, Cd et W.

9. Procédé selon la revendication 2, où le catalyseur acide solide est un catalyseur contenant un sel d'acide inorganique d'au moins un élément choisi dans le groupe consistant en Fe, Tl, Ca, Mn, Bi, Sr, Y, Al, Zn, Cd, Ni, Mg, In, Be, Co, Ga et les lanthanides.

10. Procédé selon la revendication 2, où le catalyseur métallique est un catalyseur contenant au moins un élément choisi dans le groupe consistant en Cu, Ag, Pt, Ni, Co, Fe, Ir, Os, Ru et Rh.

11. Procédé selon la revendication 9, où le sel d'acide inorganique est un sulfate.

12. Procédé selon la revendication 8 ou 11, où l'un des éléments constituants du catalyseur est Cd.

13. Procédé selon la revendication 10, où l'un des éléments constituants du catalyseur est Ag.

**Patentansprüche**

1. Verfahren zur Herstellung von Indolen oder deren Abkömmlingen, das das Umsetzen eines Anilins mit einer Verbindung, ausgewählt unter Ethanolaminen, Ethylenglykol, Propylenglykol, 1,2-Butandiol, 2,3-Butandiol, Diethylenglykol, umfaßt.

2. Verfahren, wie in Anspruch 1 beansprucht, worin die Umsetzung in Gegenwart eines festen, sauren Katalysators, eines metallischen Katalysators oder von Aktivkohle durchgeführt wird.

3. Verfahren, wie in Anspruch 1 oder 2 beansprucht, worin die Reaktion in der Dampfphase bei einer Temperatur von 200° bis 600°C durchgeführt wird.

4. Verfahren, wie in Anspruch 1 oder 2 beansprucht, worin die Reaktion in der flüssigen Phase oder einer Dampf-Flüssig-Mischphase bei einer Temperatur von 200° bis 500°C durchgeführt wird.

5. Verfahren, wie in Anspruch 1 oder 2 beansprucht, worin die Reaktion in einer Atmosphäre aus Wasserstoffgas oder einem wasserstoffhaltigen Gas durchgeführt wird.

6. Verfahren, wie in Anspruch 1 oder 2 beansprucht, worin die Reaktion in Gegenwart von Wasser oder Wasserdampf durchgeführt wird.

7. Verfahren, wie in Anspruch 2 beansprucht, worin der feste, saure Katalysator ein Katalysator ist, der ein Oxid oder Hydroxid wenigstens eines Elements, ausgewählt aus der Gruppe bestehend aus Si, Al, B, Sb, Bi, Sn, Pb, Ga, Ti, Zr, Be, Mg, Y, Cu, Ag, Zn, Cd und den Lanthaniden, enthält.

8. Verfahren, wie in Anspruch 2 beansprucht, worin der feste, saure Katalysator ein Katalysator ist, der ein Sulfid oder Selenid wenigstens eines Elements, ausgewählt aus der Gruppe bestehend aus Pd, Pt, Cr, Fe, Ni, Co, Zn, Mo, Cd und W, enthält.

9. Verfahren, wie in Anspruch 2 beansprucht, worin der feste, saure Katalysator ein Katalysator ist, der ein anorganisches Säuresalz wenigstens eines Elements, ausgewählt aus der Gruppe bestehend aus Fe, Tl, Ca, Mn, Bi, Sr, Y, Al, Zn, Cd, Ni, Mg, In, Be, Co, Ga und den Lanthaniden, enthält.

10. Verfahren, wie in Anspruch 2 beansprucht, worin der metallische Katalysator ein Katalysator ist, der wenigstens ein Element, ausgewählt aus der Gruppe bestehend aus Cu, Ag, Pt, Ni, Co, Fe, Ir, Os, Ru und Rh, enthält.

11. Verfahren, wie in Anspruch 9 beansprucht, worin das anorganische Säuresalz ein Sulfat ist.

12. Verfahren, wie in Anspruch 8 oder 11 beansprucht, worin eines der Bestandteil-Elemente des Katalysators Cd ist.

13. Verfahren, wie in Anspruch 10 beansprucht, worin eines der Bestandteil-Elemente des Katalysators Ag ist.